(19) Europäisches Patentamt
European Patent Office
Office européen des brevets

(11) Publication number: **0 314 528 B1**

# (12) EUROPEAN PATENT SPECIFICATION

(45) Date of publication of patent specification: **16.12.92**

(51) Int. Cl.⁵: **A61L 15/24**, A61L 15/44, A61K 9/70

(21) Application number: **88310237.8**

(22) Date of filing: **31.10.88**

## (54) Article for the controlled release and delivery of substances to animal tissues.

(30) Priority: **29.10.87 US 114063**

(43) Date of publication of application:
**03.05.89 Bulletin 89/18**

(45) Publication of the grant of the patent:
**16.12.92 Bulletin 92/51**

(84) Designated Contracting States:
**CH DE FR GB IT LI SE**

(56) References cited:
**EP-A- 0 196 769**

**CHEMICAL ABSTRACTS, vol. 102, no. 24, 17th June 1985, page 364, abstract no. 209447v, Columbus, Ohio, US; & JP-A-60 23 312 (NITTO ELECTRIC INDUSTRIAL CO., LTD) 05-02-1985**

**CHEMICAL ABSTRACTS, vol. 87, no. 9, 29th August 1977, page 100, abstract no. 63145t, Columbus, Ohio, US; N.J. LOWE et al.: "Anti-inflammatory properties of a prostaglandin antagonist, a corticosteroid and indomethacin in experimental contact dermatitis", & BR. J. DERMATOL. 1977, 96(4), 433-8**

(73) Proprietor: **HERCON LABORATORIES CORPORATION**
**200 B Corporate Court Middlesex Business Center**
**South Plainfield New Jersey 07080(US)**

(72) Inventor: **Franz, Thomas J.**
**Watchung New Jersey(US)**
Inventor: **Shah, Kishore R.**
**Bridgewater New Jersey(US)**
Inventor: **Kydonieus, Agis F.**
**Kendall Park New Jersey(US)**

(74) Representative: **Woodcraft, David Charles et al**
**High Holborn House 52/54 High Holborn**
**London WC1V 6SE(GB)**

**EP 0 314 528 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

## BACKGROUND OF THE INVENTION

### 1. Field of the Invention

This invention relates broadly to articles of manufacture for administration of a pharmacologically active substance, transdermally and by means of implant e.g., subdermal implant). More specifically, this invention relates to articles for the controlled release and delivery to animal tissue (including but not limited to epidermal tissue) of a composition of matter comprising at least one pharmacologically active agent with or without one or more excipients therefor and/or one or more enhancers therefor, in which at least one of the pharmacologically active agents is a causative factor in the occurrence of non-allergic or allergic contact dermatitis.

### 2. The Prior Art

Relatively intensive efforts are currently being expended in the pharmacological industry to provide for the delivery, in a controlled release manner, of a variety of pharmacologically active agents or drugs to the skin, and to mucous membranes of animals. Among the various kinds of drugs which have been described as being applied from transdermal controlled release devices are anti-inflammatory drugs which have been applied to treat existing inflammatory conditions on the skin.

Thus, for example, Japan Kokai 85/122,291 published on December 11, 1986 and abstracted at Chemical Abstracts, Volume 106, No. 125909s discloses a transdermal tape containing diclofenac sodium as the active ingredient in an anti-inflammatory and analgesic transdermal tape having a soft pressure sensitive adhesive layer. The abstract states that diclofenac sodium is readily absorbed by the skin from this tape in the presence in the tape of an organic acid, such as citric acid.

South African Patent 8410-058 and German Offenlegungsschrift 33 47 278A published on June 24, 1985 disclose prolonged percutaneous release compositions containing an active substance, preferably an anti-inflammatory agent, in an elastomer mixture consisting of diene rubbers. By the same token, South African Patent 8410-059 and German Offenlegungsschrift 33 47 277 published on December 27, 1984 disclose prolonged percutaneous release compositions containing an active substance, preferably an anti-inflammatory agent, in an elastomer mixture consisting of amorphous olefinic copolymers.

It is also known that anti-inflammatory drugs can be applied to the skin or mucosa by other means, such as by topical ointments, creams, lotions, and the like. For example, PCT Patent 87/02891 entitled "Compounds and Method for the Topical Treatment of Inflammation and Pain", discloses combinations of anti-inflammatory drugs, and, in particular, discloses that esters of methyl salicylate with certain non-steroidal anti-inflammatory drugs (NSAID's) can be used for topical administration to mammals, to elicit a response which combines the anti-inflammatory and analgesic effects of the NSAID and the counter-irritant effect of the methyl salicylate. Typical NSAID's which can be used in this way are aspirin (acetylsalicic acid), ibuprofen and indomethacin. The NSAID-methyl salicylate ester is administered in the form of a pharmaceutical composition, such as an ointment, gel, lotion, cream or the like.

Canadian Letters Patent 1,223,818 issued on July 7, 1987 and 1,223,819 issued on July 7, 1987 disclose penetrating topical pharmaceutical compositions containing enhancing ingredients in the form of N-(2-hydroxyethyl)-pyrrolidone and 1-dodecyl-azacycloheptan-2-one, respectively, to enhance the penetration into the skin of a pharmaceutically active agent. Among the many different pharmaceutically active agents which are disclosed by these Canadian patents, mention may be made of non-steroidal anti-inflammatory agents, such as salicylic acid, acetyl salicylic acid, methyl salicylate, glycol salicylate, salicylmides, benzyl-2,5-diacetoxybenzoic acid, ibuprofen, fulindac, naproxen, keto-profen, etofenamate, phenylbutazone, indomethacin, piroxicam, and mixtures thereof; steroidal anti-inflammatory agents, and, in particular, corticosteroids, such as triamcinolone acetonide, hydrocortisone acetate, betamethasone valerate, fluocinolone acetonide, flupamesone, and fluocinonide. When fluocinonide has been used in topical compositions, such as creams, it has generally been used in amounts of 0.01 to 0.1 weight percent. The Canadian patents disclose that the customary dosage levels for fluocinonide in their invention is 0.05%.

Bonney et al, Arzneim.-Forsch, 35(4), pages 715 to 720 (abstracted at Chemical Abstracts, Volume 103, No. 81418h) discloses the use of 2-(3-(1,1-dimethylethyl)5-methoxy phenyl-oxazolo(4,5-b)pyridine as a topical anti-inflammatory and analgesic compound lacking systemic activity and gastric side effects. It is indicated that this compound is as potent as indomethacin in inhibiting ultra-violet light-induced erythema in guinea pig skin and it is further indicated that this compound is an effective analgesic when applied

topically to rat footpad. It is further indicated that the compound is a cyclooxygenase inhibitor and an inhibitor of prostaglandin E2 but not leukotriene C4 synthesis.

Tsukada et al, Arzneim.-Forsch, 28(3), pages 428 to 438 discloses the anti-inflammatory, analgesic, anti-pyretic and other pharmacological effects of osepinao-(6,11-dihydro-11-oxodibenz(b,e)oxepin-3-acetic acid and compared the effects with known non-steroidal anti-inflammatory drugs such as indomethacin.

It is also known that many drugs irritate the skin when used in, for example, transdermal drug delivery systems. Such irritation results in epidermal and dermal inflammation which persists beyond the time that the patch is removed from the skin and may be symptomatic to the patient. Pharmacologically active substances which are known to cause contact dermititis are, for example, (a) guanfacine; (b) RO 22-1327, also known as (5Z, 13E, 15R, 16R)-16-fluoro-15-hydroxy-9-oxoprosta-5-dienoic acid; (c) fluphenazine; and (d) clonidine.

Chemical Abstracts, vol. 102, 1985, page 364 describes a transdermal device consisting of an adhesive formulated from natural rubber, a polyterpene resin and polybutene as an 80 micron layer on a polyester film. Elonidine and prednisolone were mixed with the adhesive composition prior to application to the polyester film. The device is stated to be effective in preventing contact dermititis when tested on rabbit skin.

Chemical Abstracts, vol. 87, 1977, page 100, describes an investigation in experimental contact dermititis. A topical preparation containing a prostaglandin antagonist, a corticosteroid and indomethacin was applied to mice having induced inflammation and decreased the erythema.

EP-A-0196769 describes a transdermal device comprising an impervious backing layer, a polymer matrix disc layer having a pharmaceutical dispersed therein and an adhesive layer, the adhesive layer containing one or more skin absorption enhancers for the pharmaceutical. The preferred polymer for the polymer disc layer is a cross-linked polysiloxane polymer.

Until now, there has not been any transdermal device which has prevented the contact irritant and contact allergic dermatitis which has arisen from the application of various pharmaceutically active agents which give rise to the dermatitis.

## SUMMARY OF THE INVENTION

According to the present invention there is provided a transdermal device for the controlled release of a pharmacologically active agent which is a causative factor of contact dermatitis and of an anti-dermatitic substance which is chemically compatible with the pharmacologically active agent and which prevents or minimises the occurrence of said contact dermatitis, characterised in that the device includes a reservoir for said pharmacologically active agent which comprises a polyvinylchloride plastisol layer having said pharmacologically active agent dispersed therein.

The anti-dermatitic substances employed in the present invention may be one or combinations of the following groups (a) corticosteroids; (b) chemicals that compete with the arachidonic acid biotransformation; (c) inhibitor substances of the arachidonic acid cascade; (d) free radical scavenger substances; (e) Vitamin E; (f) nordihydroguaiaretic acid; (g) Vitamin D; and (e) leukotriene receptor antagonists.

The devices of the present invention are in the form of transdermal controlled release systems wherein the reservoir for the pharmacologically active agent is a solid PVC plastisol.

With reference to that aspect of the present invention concerning the use of corticosteroids to prevent skin irritation associated with transdermal drug delivery, it is well known that corticosteroids are a class of drugs used for their anti-inflammatory activity. One aspect of the present invention concerns the coadministration of a corticosteroid with another drug from a transdermal patch. The coadministration of a suitably potent corticosteroid at a constant or sufficient rate blocks the inflammatory response normally caused by the other drug being delivered. A mildly irritating "other" drug only requires the use of a corticosteroid of high potency. Coadministration of the corticosteroid from the patch with the "other" drug is essential since the anti-inflammatory activity needs to be maintained at a sufficient or constant level to offset the constant or sustained delivery rate of the irritating "other drug". The "irritating other drugs" is one which is applied for an indication other than a dermatitic indication, but which causes allergic or non-allergic contact dermatitis as a result of treating the other indication.

Examples of corticosteroids useful in the practice of the present invention are set forth at pages 18 and 19 of Canadian Letters Patent 1,223,819 incorporated by reference herein and include (but are not limited to) alpha-methyldexamethasone, beta-methylbetamethasone, beclomethasone dipropionate, betamethasone benzoate, betamethasone dipropionate, betamethasone valerate, clobetasol valerate, desonide, desoxymethasone, dexamethasone, diflorasone diacetate, difluocortolone valerate, fluadrenolone, fluclorolone acetonide, flumethasone pivalate, fluosinolone acetonide, fluocinonide, flucortine butylester, fluocortolone,

fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenolone acetonide, medrysone, amcinafel, amcinafide, betamethasone and the balance of its esters, chloroprednisone, clocortelone, clescinolone, dichlorisone, difluprednate, flucloronide, flunisolide, fluoromethalone, fluperolone, fluprednisolone, hydrocortisone meprednisone, paramethasone, prednisolone, prednisone hydroxyltriamcinolone and beclomethasone dipropionate.

The amount of corticosteroid useful in she practice of the present invention, particularly when used with transdermal patches such as either in the gel example or in the polymer example vary from about 0.5% up to about 5%, such as 1 to 3% by weight of the weight of the layer being utilized for supplying to the animal tissue the corticosteroid. It has been found that the amount of corticosteroid which is to be used generally is often higher than would be expected to obtain the benefits of the corticosteroid. Thus, for example, when fluocinonide is employed the amounts generally should be 1.0% to 5%, whereas fluocinonide is normally used in topical preparations in amounts of 0.01 to 0.1%.

With reference to that aspect of the present invention concerning the use of inhibitors of the arachidonic acid cascade to prevent contact dermatitis associated with, for example, transdermal delivery, it has been determined that products of the arachidonic acid cascade are important from a pathophysiologic standpoint in a number of skin diseases in which inflammation is a prominent part. We have further determined that drugs which inhibit key enzymes in the process (e.g., phospholipase $A_2$, 5-lipoxygenase, 12-lipoxygenase, or cyclo-oxy-genase) or block the end-organ response (e.g., receptor antagonists) are effective in the treatment of inflammatory skin diseases.

As a result of the application of the present invention, it is now possible to prevent the occurrence of contact dermatitis, both irritant or allergic, through the coadministration of one or more selective inhibitors of the arachidonic acid cascade. By preventing even the possibility of contact dermatitis, certain drugs Which have been considered unsuitable for transdermal application because of this adverse reaction have now become useable.

With reference to that aspect of the present invention concerning the use of free-radical scavengers for eliminating or minimizing irritation and/or sensitization of skin associated with, for example, transdermal delivery of some drugs, it is now recognized that the irritation mechanism involves inflammatory reaction of skin caused by the products of arachidonic acid transformations in the epidermis and whole skin. Arachidonic acid is released as a result of physical, chemical or hormonal stimulation. The transformation of arachidonic acid to its metabolic products (leukotrienes, 12-hydroxyeicosatetraenoic acid and prostaglandins) which are responsible for the inflammatory reaction, is an enzyme (lipoxygenase and cyclooxygenase) catlyzed free-radical oxidative process.

An aspect of the present invention thus concerns the incorporation of one or more free-radical scavengers in the formulation of, for example, a transdermal patch for a drug known to cause skin sensitization or irritation. Upon application of the patch to the skin, the free-radical scavenger will be delivered to the skin along with the drug by the process of diffusion. The free-radical chemical transformations of arachiodonic acid to inflammatory metabolites are entirely prevented or effectively minimized by the presence in the skin of free-radical scavengers.

Some of the free-radical scavengers operable in the practice of the present invention include hindered phenols such as 2,6-ditertiarybutyl-para-cresol (BHT), p-tertiary butyl catechol, hydroquinone, benzoquinone, and N,N-diethylhydroxyamine.

With reference to that aspect of the present invention concerning the use of combinations of corticosteroids and arachidonic acid cascade inhibitors to prevent irritation and sensitization associated with, for example, transdermal delivery of "other" drugs, contact dermatitis has been found to be caused as a result of application to the skin in transdermal devices of many drugs having an irritant or allergic effect. Since both the development and expression of these reactions are complex and involve many cellular and biochemical reactions as well as mediators, blockade of these adverse reactions requires the use of more than one anti-dermatitic agent.

We have found it appropriate to use antidermatitic agents that have different mechanisms of action and affect different steps in the inflammatory cascade, e.g., membrane stabilization and lipoxygenase inhibition, or receptor blockade. Thus, a combination of a corticosteroid (as exemplified supra) and one or more inhibitors of the arachidonic acid cascade, when incorporated into, for example, a transdermal device containing an irritant "other" drug, will block the adverse reactions caused by the irritant or sensitizing other" drug. Coadministration of the two or more anti-dermatitic agents from the patch with the irritant or sensitizing "other" drug is often desirable since the anti-inflammatory activity needs to be maintained at a sufficient or constant level to offset the sustained constant delivery rate of the irritant "other" drug.

We have determined that materials similar to arachidonic acid which would be biotransformable in

similar ways to arachidonic acid compete with the arachidonic acid biotransformation and, therefore, less arachidonic acid will be transformed into products that would be irritating, such as leukotrienes and prostaglandins with four double bonds. It has further been determined that the products of the biotransformation of the chemicals of similar nature to arachidonic acid are not irritating due to the different number of double bonds or the presence of triple bonds. Such chemicals include eicosatetraenoic acid, eicosapentanoic acid and dihomolinoleic acid. We have found that other chemicals of similar chemical structure as the aforementioned three chemicals are also useful. Thus, for example, these chemicals could be delivered from the same patch as the drug causing the irritation or they can be given orally in larger amounts or in a combination thereof. The above mentioned chemicals which compete with the arachidonic biotransformation have been found to be useful in conjunction with chemicals that inhibit the steps of the arachidonic biotransformation cascade such as antioxidants, steroids and receptor antagonists.

We have further determined that irritation and sensitization caused by the specific drug applied to the skin will be minimized or eliminated by pretreatment and/or addition of the following ingredients in, for example, a transdermal delivery patch containing the drug that causes the irritation. These ingredients applied alone or in combination include, Vitamin D, nordihydroguaiaretic acid and/or Vitamin E.

As will be seen from the examples set forth in the "Detailed Description of the Drawings" section, infra. the anti-dermatitic agent which will substantially inhibit or eliminate non-allergic or allergic contact dermatitis as a result of treatment of animal tissue with a pharmacologically active agent which gives rise to the dermatitis is added from the same article as the pharmacologically active agent and, indeed, may be admixed with such pharmacologically active agent. Thus, for example, the anti-dermatitic agent may be applied from the same drug reservoir as the pharmacologically active agent is applied from; or the anti-dermatitic agent may be applied from a reservoir separate from the reservoir containing the pharmacologically active agent.

Our invention contemplates, specifically, articles for the controlled release and delivery to animal tissues, preferably animal epidermal tissues, of one or more pharmacologically active agents which are a causative factor in the occurrence of non-allergic or allergic contact dermatitis. Such an article comprises:

(a) a polymer layer (for example, a polyvinyl chloride-polyvinyl acetate layer);

(b) one or more pharmacologically active agents, at least one of which is a causative factor in the occurrence of non-allergic or allergic contact dermatitis and each of which is chemically compatible with said polymer, in intimate admixture with said polymer; and

(c) at least one anti-dermatitic substance chemically compatible with one or more of said pharmacologically active agents.

The anti-dermatitic substance has a chemical constituency different from that of the chemical constituency of any of the pharmacologically active agents or any of the excipients therefor or any of the enhancers therefor. When a surface of the polymer layer is in contact with the animal epidermal tissue to be treated with one or more of the pharmacologically active substances, one or more of the pharmacologically active substances is absorbed into and through the animal epidermal tissue and thence into the circulatory system of the animal. One or more of the pharmacologically active substances is present in such a concentration in the polymer layer that an effective amount of one or more of the pharmacologically active substances is absorbed into the animal in a prescribed period of time and normally there could occur during or after the prescribed period of time a non-allergic or allergic contact dermatitis capable of being caused by one or more of the pharmacologically active agents. In the present invention, however, the anti-dermatitic substance is present in such a concentration in the article as to prevent or minimize the occurrence of the non-allergic or allergic contact dermatitis during or after the prescribed period of time.

The enhancers which are often used to improve the penetration of drugs into the skin are themselves often a cause of contact dermatitis. When such irritating enhancers are employed, the present invention has the additional advantage that the anti-dermatitic agents used in the present invention are also effective against the enhancers. Examples of irritating enhancers against which the anti-dermatitic agents of the present invention are active include oleic acid; oleyl alcohol; linoleic acid; propylene glycol; hexylene glycol; lauramidopropyl betaine, such as sold under the tradename Mirataine BB by Miranol Chemical Co., Inc.; dimethyl sulfoxide; dimethyl formamide; dimethyl acetamide; decyl methyl sulfoxide; N-methyl pyrrolidone; ethanol; isopropyl alcohol; and t-butanol.

The anti-dermatitic agent useful in the practice of the present invention may be employed in one or more of the components of any of the transdermal devices disclosed in the following publications, which publications are incorporated by reference herein: Canadian Letters Patent 930,668; U.S. Letters Patent 3,921,636, issued on November 25, 1975; European Application 186,071 filed on December 13, 1985; U.S. Letters Patent 4, 661,105, issued on April 28, 1987; U.S. Letters Patent 4,681,584, issued on July 21, 1987; and U.S. Letters Patent 4,684,524, issued on August 4, 1987.

Excipients which are often used in transdermal devices are themselves often a cause of contact dermatitis.

Examples of excipients which are known to be causative agents of contact or allergic dermatitis and against which the anti-dermatitic agents of the present invention are useful are as follows: plasticizers, such as dioctyl phthalate and diamyl adipate; drug release control substances, such as polycaprolactone and cellulose acetate butyrate; tethermixotropic active agent compositions to enhance the stability and the dispersibility of compositions, for example, surfactants including nonionic surfactants, such as sorbitan monostearate, polysorbate 80 (polyoxyethylene (20) sorbitan monooleate), and polyoxyethylene-4-stearate; antioxidants to prevent degradation during prolonged periods of storage including 2,6-ditertiary butyl-p-cresol; propyl gallate, 6-ethoxy-1,2-dihydro-2,2,4-trimethyl-quinoline; preservatives, such as thimerosal, benzyl alcohol, benzalkonium chloride, antioxidants; such as benzoyl peroxide, sodium metabisulfite, propylgallate, surfactants; anionics such as sodium lauryl sulfate, cationics such as alkyltrimethylammonium chloride, nonionics such as polyoxytheylene(20) sorbitan monooleate, keratolytics; such as salicylic acid, lactic acid, urea.

## BRIEF DESCRIPTION OF THE DRAWINGS

Figure 1 is a cross-sectional view of a preferred pharmacologically active agent/anti-dermatitic agent delivery device useful in accordance with the practice of the present invention.

Figure 2 is a plan view of a strip material in accordance with the present invention containing pharmacologically active agents and anti-dermatitic agents viewed from the surface which is applied to the patient's skin.

Figure 3 is a plan view of material in accordance with this invention containing pharmacologically active agents and anti-dermatitic agents viewed from the surface away from the surface which is applied to the patient's skin.

Figure 4 is an enlarged sectional schematic view of a specific embodiment of a device useful in the practice of the present invention containing pharmacologically active agents and anti-dermatitic agents; a transdermal device for controlled release of a pharmacologically active agent simultaneously with controlled release of an anti-dermatitic agent from a plastisol monolayer through a membrane transdermally.

Figure 5 is an enlarged sectional schematic view of another embodiment of the invention where there is no membrane separating the plastisol monolayer from the epidermis and whereby pharmacologically active agent is transported from the plastisol transdermally into the patient simultaneously with the transport of anti-dermatitic agent.

Figure 6 is a perspective view of a roll of material in accordance with this invention containing both pharmacologically active agent and anti-dermatitic agent having a chemical constituency different from the chemical consistency of the pharmacologically active agent.

Figure 7 is a fragmentary enlarged cross-sectional view depicting component elements of a transdermal delivery system of a transdermal patch containing pharmacologically active agent and an anti-dermatitic agent having a chemical constituency different from the pharmacologically active agent.

Figures 7A, 7B, 7C, 7D and 7E are fragmentary enlarged cross-sectional views of several embodiments of component elements of a transdermal delivery system containing pharmacologically active agent and anti-dermatitic agent, with the chemical constituency of the pharmacologically active agent being different from the chemical constituency of the anti-dermatitic agent.

Figure 8 is a cross-sectional view illustrating an embodiment of a transdermal patch containing pharmacologically active agent and anti-dermatitic agent, the chemical constituency of the anti-dermatitic agent being different from the chemical constituency of the pharmacologically active agent.

Figure 9 is a cross-sectional view illustrating another embodiment of a transdermal patch containing pharmacologically active agent and anti-dermatitic agent, the chemical constituency of the anti-dermatitic agent being different from the chemical constituency of the pharmacologically active agent.

Figure 10A is a medical bandage seen in open structure with the bandage defining a means for placing the bandage on the patient, the bandage containing a pharmacologically active agent, the chemical constituency of the pharmacologically active agent being different from the chemical constituency of the anti-dermatitic agent.

Figure 10B is a schematic cross-sectional representation of a controlled transdermal nitrogylcerin delivery system for delivering nitroglycerin at high transdermal fluxes, and containing at least one of its components an anti-dermatitic agent different in chemical structure from the chemical structure of the pharmacologically active agent.

Figure 11 is an open view of a dispenser designed with means for forming a releasing passageway in

operation in a fluid environment of use, containing a pharmacologically active agent in its internal space and admixed therewith an anti-dermatitic agent, the anti-dermatitic agent having a chemical constituency different from the pharmacologically active agent.

Figure 12 is the infra-red spectrum of ADMEX"760 as used in Example I, infra.

Figure 13 is a schematic diagram of an arachidonic acid transformation cascade.

Figure 14 is a schematic diagram showing biosynthesis of the products of arachidonic acid.

## DETAILED DESCRIPTION OF THE DRAWINGS

Referring to Figure 1, the device 1 includes a solid plastisol monolayer 2 which is composed of or comprises resin, plasticizer, a pharmacologically active agent as set forth supra and an anti-dermatitic agent.

A formulation for layer 2 may comprise from about 20 to about 70% by weight of the vinyl resin, from about 20 up to about 70% by weight of plasticizer composition, from about 0.5 up to about 35% by weight of pharmacologically active agent and the remainder being other excipients, such as materials which will enhance skin penetration, for example AZONE$^R$ having the structure:

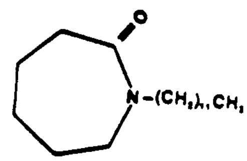

which happens to act both as a plasticizer and as an agent which enhances transdermal penetration of pharmacologically active agents. Such pharmacologically active agents include clonidine, or RO 22-1327 also called (5Z,13E,15R,16R)-16-fluoro-15-hydroxy-9-osoprosta-5-dienoic acid, guanfacine hydrochloride, guanfacine, fluphenazine, trifluoroperazine, and timolol.

The articles of the present invention include but are not limited to transdermal devices and comprise, for example, a polyvinyl chloride plastisol layer in a fused state and a pharmacologically active agent uniformly dispersed in the fused layer which may be referred to as a reservoir for the pharmacologically active agent. The articles of the present invention also include polyvinyl chloride layers which are in a gelled state, with the pharmacologically layer being uniformly dispersed in the gel layer. When a gelled vinyl layer is employed, an organic, non-volatile gel forming additive is added in an amount sufficient to form a gel. The gel forming additive can be, for example, isopropylpalmitate, isopropyl myristate, mineral oil, silicone oil, soybean oil and the like, and can be present in an amount of 5 to 30% by weight based on the total weight of the layer.

An anti-dermatitic agent may be admixed with the pharmacologically active agent or the anti-dermatitic agent may be in a layer separate from the pharmacologically active agent. When using polyvinyl chloride, the polyvinyl chloride reservoir may be prepared from polyvinyl chloride resin and a primary plasticizer for the resin.

When using polyvinyl chloride, the polyvinyl chloride resin employed in the practice of the present invention can be any of the PVC resins which are known in the art and commercially available. A preferred grade of polyvinyl chloride resin employed in the practice of the present invention is that which is specifically used in preparing PVC plastisols, namely PVC resins which are made by the well known emulsion polymerization process, which are hard spheres of particle size between 0.05 and 20 microns, such as between 1 and 20 microns, for example, between 1 and 5 microns, or between 0.05 and 1 micron, and which do not have the ability to absorb plasticizers to any great extent. Instead, the plasticizer wets the resin particles at room temperature and only then very slowly penetrates and solvates the resin. These PVC resins when mixed with plasticizers, such as a mixture of 30% primary plasticizer, 70% PVC resin, give a flowable liquid known as plastisol which can then be fused at, for example, approximately 121 °C (250 °F) for approximately 30 seconds to provide a solid polymer layer.

The PVC resin employed in the present invention can also be a general purpose PVC resin which is produced by suspension or bulk (mass) polymerization and which is used in calendering and extrusion processes, which is 50 to 200 microns, such as 100 to 150 microns in diameter, and is like sponges. Thus, the general purpose resins are capable of absorbing large amounts of plasticizers so that even a 50% DOP and 50% PVC resin would result in a non-flowing solid material.

The molecular weight of the PVC resins employed in the present invention preferably is a weight

average molecular weight between 80,000 and 250,000, such as a weight average molecular weight of 123,000. A suitable polyvinyl chloride resin is one sold by Occidental Chemical Co. under the designation FPC 6338 containing about 96% vinyl chloride monomer units of about 4% vinyl acetate monomer units. Thus, the polyvinyl chloride resin can be a copolymer containing preferably at least 90% by weight vinyl chloride monomer units, such as a copolymer based on vinyl chloride and vinyl acetate.

The polyvinyl chloride resin generally is present in the layer in an amount of 20 to 75 weight percent, preferably 20 to 70 weight percent, based on the total weight of the vinyl plastisol composition.

The primary plasticizer which can be employed in the present invention can be dioctylphthalate (DOP), benzylbutylphthalate, tri-2-ethylhexylmalaete, dioctyl adipate, epoxidized soybean oil, polymeric adipate plasticizers, which are polymers of adipic acid with a monomer, such as propylene glycol, and for example can be obtained under the designation Drapex 334F from Witco Chemical Corp., or any other primary plasticizer for PVC which is biologically acceptable.

Other examples of polyester adipates, glutarates and sebacates which can serve as plasticizers are polyester adipate P-644; polyester glutarate P-530; polyester glutarate P-540; polyester glutarate P-550; polyester glutarate P-7035; polyester glutarate P-7035M; polyester glutarate P-7046; polyester glutarate P-7092; and polyester glutarate P-1070 manufactured by the C.P. Hall Co., 7300 South Central Avenue, Chicago, Illinois 60638. Other preferred plasticizers are those which are known as "adipate: plasticizers, for example, ADMEX$^R$760 which is a high molecular weight (MW = 8000) adipate plasticizer manufactured by the Sherex Division of Nuodex Inc., and having an infra-red spectrum as set forth in Figure 12. In general, polyester plasticizers which are polyesters of (i) 1,4-terephthalic acid and/or 1,2-phthalic acid with (ii) ethylene glycol or 1,3-propylene glycol having molecular weights in the range of 4000 to 10,000 are preferred.

Another preferred plasticizer Which also acts as a skin penetrating enhancer for pharmacologically active drugs which are intended for transdermal delivery from devices such as those set forth in Figures 1, 5 and 6 is the compound known as AZONE$^R$ marketed by the Nelson Research and Development Co.

Mixtures of known plasticizers can be used. The term "primary plasticizer" as used herein refers to a plasticizer which can be used alone to effect plasticization and is highly compatible with PVC at high concentrations, such as, for example, 150 parts per hundred. Primary plasticizers are contrasted with "secondary plasticizers" which, because of limited compatibility with PVC, cannot be used alone. See, Kirk-Othmer Encylopedia of Chemical Technology, Volume 23, 3rd Edition, especially pages 913 and 914 for a discussion of primary and secondary plasticizers, which is incorporated by reference herein.

The primary plasticizer generally is present in an amount of 20 to 85 weight percent, preferably 20 to 70% based on the total weight of the vinyl plastisol layer.

The PVC plastisol may optionally contain other additives or "excipients" useful in the practice of this invention, for example, materials which enhance skin penetration of the pharmacologically active substances (e.g. 1,6-hexanediol and n-decyloleate) and thickeners, e.g. silica (preferably "fumed" silica, for example AEROSIL$^R$ in an amount of from 1 to 6% of the layer).

With reference to Figure 1, a blended plastisol containing, for example, PVC, DOP, fluphenazine and fluocinonide is coated at a rate of about 1.22 kg/m$^2$ (36 ounces/yd$^2$) on a backing and then fused into solid plastisol layer 2. The backing may be a single layer of drug impermeable plastic to other material, but is preferably composed of two layers 3 and 4. Layer 3 may be MYLAR$^R$ (polyester produced from ethylene glycol and phthalic anhydride) about 12.7 microns (0.5 mils) thick, and layer 4 may be PVC, about 102 microns (4 mils) thick. The backing 3, substantially blocks loss of drug and anti-dermatitic agent from the plastisol layer 2 other than in the direction of the surface which, in use, will contact the patient's skin.

The blended plastisol which is coated on the backing can be fused into a homogeneous solid by heating it for a short period, such as 15 to 30 seconds, at a temperature of, for example, 121 to 138 °C (250 to 280 °F). The use of a plastisol to form solid layer 2 enables layer 2 to be formed by using a low temperature for a short period of time, and provides conditions which do not affect the stability of the pharmacologically active agents.

A strip of solid plastisol layer 2 and backing 3, 4 is then bonded to a pressure-sensitive adhesive layer 5 which in turn is provided with a non-adhesive backing 6 such as one made of plastic, moisture-proof fabric, aluminium foil, etc.

When not in use, the entire surface intended for skin contact is preferably covered with a release paper 7 or the like which is removed to expose surfaces of the adhesive layer 5 and drug containing plastisol layer 2 for application to the patient's skin.

Figure 2 shows a plan view of the strip of material 20 during the stage of manufacture at which a strip of plastisol 2 (backing 3,4 not shown) has been applied to the adhesive tape (backing 6 not shown). For the preferred device for the controlled administration of pharmacologically active agent, e.g. drug RO 22-1327,

taken further together with an anti-dermatitic agent such as a plastisol strip preferably about 25 mms (one inch (1")) in width is applied on a 63.5 mms (two and one-half inch (2-1/2")) wide pressure-sensitive adhesive strip.

Figure 3 shows a plan view of a strip of the material 30 in accordance with the present invention; spaced lines 31 may be embossed or printed on the surface away from skin contact so that the patient may conveniently measure out and cut off the proper amount of the tape device to provide the prescribed daily dosage. For a device for administering the pharmacologically active agent, e.g. RO 22-1327 for example, a segment 25 mms (1") long cut from the longer tape (resulting in an approximately 645 $mm^2$ (one square inch (1 sq. inch)) of active surface against the patient's skin) will provide a dosage of about 17 mg/24 hours (along with the anti-dermatitic drug, e.g. fluocinonide).

Referring to Figure 4, there is shown a transdermal device 33 comprised of a backing layer 34, a reservoir layer 35 that contains supplies of percutaneous absorption enhancer, pharmacologically active substance, such as fluphenazine, an anti-dermatitic agent, such as fluocinonide, a diffusion membrane layer 36, and a peripheral ring 37 of contact adhesive. Diffusion membrane layer 36 may be composed of a polymer, such as a copolymer of ethylene and methyl acrylate with the methyl acrylate being in the range of 2 to 90% by weight of the polymer, or blends of such copolymer with low density polyethylene, high density polyethylene or linear low density polyethylene. The contact adhesive component of device 33 is in the form of a peripheral ring 37. Optionally, backing layer 34 may also be a semi-permeable membrane. Neither the pharmacologically active agent, the anti-dermatitic agent nor the enhancer passes through ring 37, and therefore ring 37 need not be permeable to those compositions. Optionally, the contact adhesive may be attached directly to the membrane 36 in which case the adhesive may be attached directly to the membrane 36 in which case the adhesive is selected so that it is permeable to the pharmacologically active agent and, in addition, to the anti-dermatitic agent.

The base surface from which the pharmacologically active substance, the anti-dermatitic agent and the enhancer (e.g.. AZONE", is transferred to the skin is defined by diffusion membrane layer 36. Backing layer 34 is not flat but, instead, forms a pocket or cavity in which the reservoir layer 35 is held. The outer edge of backing layer 34 is sealed to the peripheral ring 37 of the contact adhesive as more specifically set forth in U.S. Letters Patent 4, 379,454 issued on April 1, 1983, the disclosure of which is incorporated herein by reference. Similarly, an article within the contemplation of the present invention may have a matrix as is illustrated in Figure 5 wherein the backing 61 totally surrounds the PVC-platisol-plasticizer matrix 62 and is firmly in place as with an adhesive on the skin 63. The anti-dermatitic agent may also be included in the adhesive.

As shown in Figure 6, the device of the invention may conveniently be provided in the form of a tape roll 50 from which daily dosage requirements of the pharmacologically active agent along with the anti-dermatitic agent may be clipped by the patient.

The device is capable of application to humans or other animals capable of usefully absorbing pharmacologically active agents through the skin.

Other embodiments of the present invention similar to these set forth in Figures 1 to 6, supra, are those useful in, for example, U.S. Letters Patent 4,573,996 and 4,573,995 issued on March 4, 1986, the specifications for which are incorporated by reference herein.

Referring to Figures 7, 7A, 7B, 7C, 7D, 7E, 8 and 9, there is shown a transdermal delivery system 110 which comprises an impermeable backing member 111, a drug reservoir member 112 consisting of a pharmacologically active agent in admixture with anti-dermatitic agent 113 dispersed in carrier 114 and a rate controlling member 115, said system which together with a means to attach the system to the skin forms a transdermal patch or bandage. The rate controlling member may be (a) a microporous membrane 115a as seen in Figure 7A, (b) a diffusion controlling membrane 115b as seen in Figure 7B, (c) a rate controlling adhesive 115c as seen in Figure 7C (which also may contain anti-dermatitic agent or which may contain anti-dermatitic agent), (d) a combination of a microporous membrane 115a and an adhesive layer having rate controlling properties 115c which together perform the rate controlling function as seen in Figure 7D, or (e) may be a combination of a diffusion controlling membrane 115b and a rate controlling adhesive layer 115c as seen in Figure 7E.

When an adhesive is contributing to or performing a rate controlling function. the adhesive in these instances is performing a dual function of rate control and of attaching the transdermal delivery system to the skin. The adhesive as stated supra may also contain the anti-dermatitic agent which diffuses to the skin simultaneously with the diffusion of the pharmacologically active agent.

Figure 8 depicts an embodiment of a transdermal patch with attachment means 116 in which the drug reservoir 112 is a solid with the drug reservoir also including the anti-dermatitic agent.

Figure 9 depicts an embodiment in which the drug reservoir 112 is a semi-solid or ointment wherein the

face of the backing member 111 contiguous to the drug reservoir is joined at the edges to the face of the microporous membrane 115 contiguous to the drug reservoir. The drug reservoir 112 contains in addition to pharmacologically active agent, anti-dermatitic agent. The edges are joined preferably by heat sealing but also may be sealed by crimping, using sealants and by means for effecting a seal.

The impermeable backing member 111 of Figures 7 to 9 is preferably of a polyester occlusive film. Other materials suitable for a backing include foil, polyethylene coated foil, polyethylene, Mylar polyester, polypropylene and polyvinyl chloride.

The drug/anti-dermatitic agent reservoir 112 can be a dispersion of the pharmacologically active agent such as clonidine and anti-dermatitic agent, such as fluocinonide, in a carrier.

The carrier may be a solid. i.e., of a non-mobile or non-flowable material or may be of a semi-solid preparation often referred to as ointments. Suitable semi-solid carriers include gelled mineral oil. e.g., mineral oil gelled with 5% polyethylene (commercially available as Plexi Gel 50W from Parke Davis), polyisobutylene, aluminum stearate or even propylene glycol or fatty acid esters. When propylene glycol is used, the propylene glycol also can in many instances cause allergic or contact dermatitis. In this case, the anti-dermatitic agent acts against such causation of contact or allergic dermatitis. Solid carriers include silicone, acrylic adhesive, plasticized polyvinyl chloride and the like. By "drug reservoir composition" is meant a pharmacologically active agent taken alone or taken in conjunction with an anti-dermatitic agent in one of the aforementioned carriers.

Membrane 115a of Figures 7A and 7D is a microporous membrane which may be of any porous material permitting the passage of the pharmacologically active agent and the anti-dermatitic agent and is inclusive of microporous polypropylene, microporous nylon, microporous polycarbons and the like. The microporous membrane layer may be a single layer or may be of multiple layers of selected microporous materials which have been laminated together. The pharmacologically active agent taken further together with the anti-dermatitic agent passes through the micropores which are filled with mineral oil or other carrier material during the fabrication. The control effected by the microporous membrane is not by the dissolution of the drug in the membrane material but merely in travel through the pores.

Membrane 115b of Figures 7B and 7E is a diffusion controlling membrane which may be of materials in which the pharmacologically active agent dissolves and is inclusive of silicone, ethylene vinyl acetate and polyurethane.

When the rate controlling member includes an adhesive rate controlling member 115c as in Figures 7C, 7D and 7E, the preferred adhesive is selected from a class of rubber based adhesives. Other adhesives include suitable medical grade adhesives which permit migration. Examples of such adhesives include polymers of esters with acrylic acid, copolymers of the esters with other acrylic derivatives, such as acrylic acids, acrylamides, elastomeric silicone polymers, vinyl polymers, such as polyvinyl alcohols, polyvinylpyrrolidones, polyvinyl acetates, blends of cellulose derivatives and natural gums, such as guar gum, pectin and the like. When the adhesive is to be employed solely as attachment means for a patch, an adhesive having no rate-controlling properties is employed. Generally, such adhesives are acrylate based adhesive systems.

The rate of travel of the pharmacologically active agent taken further together with anti-dermatitic agent is controlled by several factors, the porosity or diffusion coefficient of the pharmacologically active agent in the membrane, the porosity or diffusion coefficient of the anti-dermatitic agent in the membrane, the thickness of the membrane or the rate controlling adhesive, the concentration of the anti-dermatitic agent in the adhesive and the solubility of the pharmacologically active agent as well as the anti-dermatitic agent in the carrier material of the drug reservoir.

Referring to Figure 10A, which is a cross-sectional view of a medical bandage useful in the practice of the present invention, the bandage 210 comprises a backing member 211 that defines the top of bandage 210. Backing member 211 serves as a protective cover for bandage 210 and it imparts structural support to the bandage and it substantially keeps components, e.g., anti-dermatitic agent and pharmacologically active agent in bandage 210 from escaping the bandage. Backing member 211 is made from a material 212 that is substantially impermeable to the components in bandage 210 or member 211 is made from a combination of materials, such as a composite or a laminate to yield a backing member that is substantially impermeable to the passage of components in bandage 210.

A reservoir 213 containing anti-dermatitic agent and pharmacologically active agent adjacent to backing member 211 is positioned immediately below and in contact with one surface 214 of backing member 211. Reservoir 213 bears on its surface distant from backing member 211 a membrane 216 for controlling the release of pharmacologically active agent and anti-dermatitic agent 217 represented by dots from medical bandage 210. In the bandage outer edges 218 of backing member 211 will overlay edges 219 of reservoir 213 and they will be joined along these parameters in a fluid tight arrangement. This sealed reservoir is

effected by pressure, fusion, adhesion or through an adhesive applied to the edges of the membrane. In this structure reservoir 213 is contained wholly between backing member 211 and release rate controlling membrane 216 and reservoir 213 does not in this manufacture have any exposed surfaces. In a preferred embodiment, backing member 211 and release rate controlling membrane 216 will be inherently sealable to each other or they will include a sealing means such as a film position between and sealable to both the backing member and the release rate controlling membrane or by a layer of adhesive. Reservoir 213 comprises a continuous phase as represented by wavy line 221 and it is formed of a fluid to viscous material permeable to the passage of the anti-dermatitic and pharmacologically active agent composition 217. Reservoir 213 contains a rheological agent 222 represented by dashes. Reservoir 213 contains a dosage unit amount of pharmacologically active agent 217 that is supplied to release rate controlling membrane 216 throughout the medical history of bandage 210. The dosage amount comprises a supply of pharmacologically active agent for one hour, for eight hours for a normal night sleep, for twenty-four hours applied once daily, for forty-eight hours or longer together with an appropriate amount of anti-dermatitic agent to last as long as the supply of pharmacologically active agent lasts. In practicing the therapeutic method of drug administration, a single bandage can be on the skin, more than one medical bandage can be on the skin and the medical bandage can be applied topically successively for the intended result.

Rate controlling membrane 216 has one surface in contact with reservoir 213. Membrane 216, adjacent to reservoir 213, is formed of a material that is dense or microporous and it is a polymeric material that controls the rate of pharmacologically active agent/anti-dermatitic release from reservoir 213. Membrane 216 permits the passage of pharmacologically active agent/anti-dermatitic agent 217 at a rate dependent on the solubility of pharmacologically active agent and anti-dermatitic agent therein as well as on the thickness of the membrane. The dosage rate per area of medical bandage 210, or the flux of pharmacologically active agent/anti-dermatitic agent thus is controlled to the exterior of the bandage by regulating the composition, thickness of membrane 216 and the diffusion coefficient of the anti-dermatitic agent and the diffusion coefficient of the pharmacologically active agent. Medical bandage 210 can be with the same surface area and having different dosage of pharmacologically active agent/anti-dermatitic agent release by varying the characteristics of membrane 216. Diffusion coefficients can be determined by standard techniques.

Medical bandage 210 further comprises a layer or lamina of an adhesive 223 in contact with the releasing surface of membrane 216, that is, it is directly below and adjacent to membrane 216 or optionally adhesive 223 extends around the outer parameter of membrane 216. Contact adhesive layer 223 is the presently preferred means by which bandage 210 in this particular embodiment is affixed to a warm-blooded animal, mainly to the area of the skin selected for receiving the pharmacologically active agent (together with the anti-dermatitic agent). The composition and the thickness of adhesive layer 223 are such that layer 223 does not constitute a significant permeation barrier to the passage of pharmacologically active agent and anti-dermatitic agent and it should preferably be substantially more permeable to the passage of anti-dermatitic agent and pharmacologically active agent than membrane 216, and it is at least as permeable to pharmacologically active agent and anti-dermatitic agent as membrane 216. The adhesives used for the present purpose are dermatologically acceptable and they permit the bandage to be easily removed from the skin after the period of administration of pharmacologically active agent and anti-dermatitic agent.

Medical bandage 210 may also include a release liner 224 in contact with adhesive layer 223. Release liner 224 protects the bandage and just prior to use, it is pulled away from adhesive layer 223 and discarded. Release liner 224 is made from a material that is substantially impermeable to the passage of anti-dermatitic agent as well as pharmacologically active agent. The same material used for backing member 211 may be used to make release liner 224 provided they are strippable materials and compatible with medical bandage 210. In a preferred embodiment, the release liner is made with a pull tab to facilitate removal of the liner from bandage 210 before use.

Components of the embodiment of the present invention as set forth in Figure 10A are specifically set forth in U.S. Letters Patent 4, 661,105 issued on April 28, 1987, the specification for which is incorporated by reference herein.

Referring to Figure 10B, the system 401 is preferably fabricated in the form of a laminated pouch formed from an impermeable backing 403 bonded at its periphery to and spaced apart at its central portion from a pharmacologically active agent/anti-dermatitic agent release rate controlling membrane 404 which is coated with a contact adhesive 405 provided with a protective removable liner 406 intended to be stripped from the device prior to use. Although the preferred embodiment illustrated herein shows the use of an in-line adhesive 405, other means for holding the system in pharmacologically active agent, anti-dermatitic agent and permeation enhancer transmitting relationships to the skin include circumferentially located adhesives, adhesive overlays, belts, buckles, elastic bands or the like. The pouch is filled with a

composition 407 which comprises the pharmacologically active agent, the anti-dermatitic agent and permeation enhancer reservoir preferably in the form of a viscous gel or paste. Reference is made to U.S. Letters Patent 4,681,584 issued on July 21, 1987 and U.S. Letters Patent 4,144,317, the specifications for which are incorporated by reference herein.

Figure 11 depicts another embodiment of the article of the present invention, dispenser 310. Figure 11 is an open view of dispenser 310. In Figure 11, dispenser 310 comprises body 311, wall 312, internal compartment 314, thermo-responsive composition 315 containing pharmacologically active agent in admixture with anti-dermititic agent 316 and osmotically effective solution producing means 317. In Figure 11, wall 312 of dispenser comprises a releasing means 313 formed of a microporous composition. In this embodiment, a section of wall 312 contains a pore forming agent that is removed from wall 312 in the environment to form a pore of controlled release dimensions or in another embodiment wall 312 is formed in a part of a microporous composition comprising a plurality of micropores of precontrolled dimensions. In Figure 11, wall 312 comprises also in at least a part of section 318 formed of a composition permeable to the passage of fluid and substantially impermeable to the passage of osmotically effective compound 317. In Figure 11, thermo-responsive composition 315 containing pharmacologically active agent/anti-dermatitic agent formulation 316 is immediately adjacent to the interior surface of microporous releasing means 313 for its passage through the pores. Microporous pharmacologically active agent/anti-dermatitic agent releasing surface is an additional dispensing advantage provided by this aspect of the invention, as it functions like a diffuser for diffusing the agent over a larger agent receiving surface. This action of presenting the agent over a broad tissue area lessens the incidence of tissue irritation associated with tissue irritating agents; and the incidence of such irritation is substantially dispensed with now by the additional use of the anti-dermatitic agent as exemplified in Example I, infra.

Referring to Figure 13, there is shown the arachidonic acid (AA) transformation cascade. The two dashed lines indicate dietary sources of AA or dietary supplements (EPA) that can compete with AA for transformation. EPO indicates evening primrose oil, LIN oil; linseed oil; LA, linoleic acid; GLA, gamma-linolenic acid, DGLA, dihomo gamma-linolenic acid; PG, prostaglandin; HPETE, hydroperoxyeicosatetraenoic acid; HETE, hydroxyeicosatetraenoic acid; EAA, esterified arachidonic acid; EPA, eicosapentaenoic acid; LT, leukotriene; ETYA, eicosatetraenoic acid; TX, thromboxane; NSAID, nonsteroidal anti-inflammatory drug: NGDA, nordihydroguaiaretic acid; BW, Burroughs Wellcome; SRS, slow-reacting substance Numerals (e.g. 20:3) indicate number of carbon atoms to left of colon and number of double bonds (i.e., degree of unsaturation) to right of colon.

The substance of this figure is more fully described in Arch. Dermatol, Volume 119, July 1983 at page 541 entitled "Editorial/Leukotrienes and Other Lipoxygenase Products in the Pathogenesis and Therapy of Psorasis and Other Dermatoses" incorporated by reference herein.

Figure 14 is a diagram setting forth biosynthesis of the products of arachidonic acid. Two major routes of metabolism of arachidonic acid are shown. The lipoxygenase pathway leads to HPETE and HETE; the cyclooxygenase pathway leads to the cyclic endoperoxides (PGG and PGH) and the subsequent metabolic products. Compounds such as aspirin and indomethacin inhibit both pathways. The biosynthesis is more fully described in Chapter 28, "Prostaglandins, Prostacyclin, and Thromboxane $A_2$" by Salvador Moncada, Roderick J. Flower and John R. Vane in the text entitled "The Pharmacological Basis of Therapeutics", 7th Edition (Goodman and Gilman's), published by the Mcmillin Publishing Company, 1985, incorporated by reference herein.

## EXAMPLE I

In the examples, which follow, the following definitions apply:

RO 22-1327 is the compound called (5Z,13E,15R,16R)-16-fluoro-15-hydroxy-9-osoprosta-5-dienoic acid.

"Macule" is a well-demarcated alteration in the color of the skin without elevation or depression.

"Papule" is a well-demarcated, solid elevation of the skin with no clearly discernible visible fluid.

The Grading System for this example is as follows:

- +/- faint, or spotty, pink color within test site;
- 1 uniform pink color covering all of test site;
- 2 uniform pink to red color covering all of test site;
- 3 uniform bright red color covering all of test site;
- 4 uniform bright red color covering all of test side with edema, blisters, or weeping.

## EXAMPLE I (a)

PROTOCOL - RO 22-1327

Objective - To block the contact irritant dermatitis observed following application of RO 22-1327 patches to the skin through the incorporation of fluocinonide in the patch.

Materials - One formulation of the antihypertensive drug RO 22-1327 was used, in which either 0%, 0.1%. 1.0% or 5% fluocinonide was incorporated.

Methods - Four patches containing RO 22-1327 and fluocinonide at either 0%, 0.1%, 1.0%. or 5.0% were applied to the upper arms (two per arm) of a single human subject not previously exposed to this drug. The patches were removed at 8 hours and the sites observed for signs of dermatitis immediately and 16 hours later.

In a second test. one RO 22-1327 patch containing 1.0% fluocinonide was applied to the upper arm of the same human subject as above and removed after 24 hours. The site was examined immediately for signs of dermatitis.

Results - The incorporation of 1.0% fluocinonide into the RO 22-1327 patch resulted in a significant reduction in the irritant dermatitis associated with the use of RO 22-1327.

## Contact Dermatitis to RO 22-1327 (L472-17)

### Test 1

| Fluocinonide Concentration | 8 Hour Grading | 24 Hour Grading |
|---|---|---|
| 0 | 3+ * | 2+ |
| 0.1% | 3+ * | 1+ |
| 1.0% | +/- | 0 |
| 5.0% | 1+ | +/- |

----------------

*erythema extended outside of patch area.

### Test 2

| Fluocinonide Concentration | 24 Hour Reading |
|---|---|
| 1.0% | 2+ erythema limited to area within patch |

**EXAMPLE I (b)**

PROTOCOL - GUANFACINE

Objective - To block the contact irritant dermatitis observed following application of guanfacine patches to the skin through the incorporation of hydrocortisone in the patch.

Materials - Two formulations of the antihypertensive drug guanfacine were used in this study. Into each was incorporated hydrocortisone at either 0.5% or 2.0%. Control patches containing no hydrocortisone were also used.

Thus, the following formulations were prepared:

| | Formulation #L438-18W (Parts by Weight) | Formulation #438-18c3 (Parts by Weight) |
|---|---|---|
| Guanfacine HCl | 29.7 | 29.6 |
| Hexylene Glycol | 7.9 | 13.8 |
| Polyethylene Glycol 400 | 0.0 | 7.9 |
| Mirataine BB | 2.0 | 0.0 |
| Dioctyl Phthalate | 33.6 | 26.1 |
| Vinyl Chloride/Vinyl Acetate Copolymer (Containing 96% repeating units of vinyl chloride and 4% repeating units vinyl acetate) | 23.7 | 21.2 |
| Aerosil | 3.1 | 1.4 |

(To make the 0.5% hydrocortisone formulations, 20 mg hydrocortisone was added to 4 grams of the above formulations. To make 2.0% hydrocortisone formulations, 80 mg of hydrocortisone was added to 4 grams of the above two formulations).

Methods - Three patches of each of the two guanfacine formulations, containing either 0%, 0.5% or 2.0% hydrocortisone, were applied to the upper arm of a single human subject not previously exposed to this drug. The patches were removed after 23 hours and the sites observed for signs of dermatitis. One week later, the sites were again observed.

Results - The results are given in the accompanying table. Signs of irritant dermatitis were observed at both the 23 hour and 1 week observation period when no hydrocortisone was incorporated into the patch. No adverse reactions were seen with the 2.0% hydrocortisone patches and the results were variable with 0.5% hydrocortisone patches.

CONTACT DERMATITIS TO GUANFACINE

IMMEDIATE REACTION (23 HOURS)

| Hydrocortisone Concentrations | Formulation #L438-18w | Formulation #L438-18c3 |
|---|---|---|
| 0% | 2+ | 2+ |
| .5% | +/- | 0 |
| 2.0% | 0 | 0 |

DELAYED REACTION (1 WEEK)

| Hydrocortisone Concentrations | Formulation #L438-18w | Formulation #L438-18c3 |
|---|---|---|
| 0% | 4+* | 4+* |
| .5% | 4+* | 0 |
| 2.0% | 0 | 0 |

---------------

*edematous (i.e., fluid containing) plaque, almost microvesicular in appearance.

EXAMPLE I (c)

PROTOCOL - FLUPHENAZINE

Objective - To block contact dermatitis observed following application of fluphenazine patches to the skin through incorporation of fluocinonide in the patch.

Materials - One formulation of the anti-psychotic drug fluphenazine was used in which either 0%, 1% or 5% fluocon准nide was incorporated.

Thus, the following formulations were prepared:

| | Formulation L-473-9 (parts by weight) | Formulation L-473-31(1) (parts by weight) | Formulation L-473-31(2) (parts by weight) |
|---|---|---|---|
| Fluphenazine | 35 | 35 | 35 |
| ADMEX$^R$760 | 30 | 30 | 30 |
| Resin (96% vinyl chloride repeating monomeric units and 4% vinyl acetate repeating monomeric units) | 35 | 34 | 30 |
| Fluocinonide | 0 | 1 | 5 |

Methods - Three patches containing fluphenazine and fluocinonide at either 0%, 1% or 5% of fluocinonide were applied to the upper arm of a single human subject previously sensitized to fluphenazine. The patches were removed at 48 hours and the sites observed for signs of allergic dermatitis immediately and at 72 and 96 hours.

Results - The incorporation of l% or 5% fluocinonide almost completely blocked the allergic reaction (erythema and blistering) caused by fluphenazine.

The results are as follows:

| Percent Fluocinonide | Time of Grading | | |
|---|---|---|---|
| | 48 Hours | 72 Hours | 96 Hours |
| 0% | 4+ | 4+ | 4+ |
| 1% | +/- | 0 | 0 |
| 5% | 1+ | +/- | +/- |

**EXAMPLE I (d)**

PROTOCOL - CLONIDINE

Objective - To block the contact allergic dermatitis which occurs following application of clonidine patches to the skin through incorporation of fluocinonide in the patches.

Materials - One formulation of the anti-hypertensive drug clonidine was used in which either 0% or 2% fluocinonide was incorporated. A second formulation containing no clonidine was also tested.

Thus, the following formulations were prepared:

| | M0617C1/12 (parts by weight) | L477-52B (parts by weight) | Placebo L1407CLB/161 (parts by weight) |
|---|---|---|---|
| Clonidine | 10 | 10 | 0 |
| ADMEX[R]760 | 70 | 68 | 70 |
| Resin (96% vinyl chloride repeating units and 4% vinyl acetate repeating units) | 20 | 20 | 30 |
| Fluocinonide | 0 | 2 | 0 |

Methods - Two patches containing clonidine and either 0% or 2% fluocinonide were applied to the upper back of a single human subject previously sensitized to clonidine. A third patch containing no clonidine and no fluocinonide was also applied as a placebo. The patches were removed after 7 days and the sites read immediately and on the 8th day as well.

Results - The incorporation of 2% fluocinonide significantly reduced the allergic reaction (erythema and blistering) caused by clonidine.

The results are as follows:

| | Time of Grading | |
|---|---|---|
| Fluocinonide | 7 days | 8 days |
| 0% | 4+ | 4+ |
| 2% | 1+ | 1+ |
| Placebo | 0 | 0 |

**EXAMPLE I (e)**

PROTOCOL - TRIFLUOPERAZINE

Objective - To block the contact allergic dermatitis which occurs following application of trifluoperazine patches to the skin through the incorporation of fluocinonide in the patches.

Materials - One formulation of the tranquilizer drug trifluoperazine was used in which either 0% or 2% fluocinonide was incorporated.

Thus, the following formulations were prepared:

| | L426-44 #88-9 (Parts by weight) | L426-49-2 (Parts by Weight) |
|---|---|---|
| Trifluoperazine | 30% | 30% |
| ADMEX[R]760 | 35% | 34.5% |
| Resin (96% repeating units of vinyl chloride and 4% repeating units of vinyl acetate) | 35% | 33% |
| Fluocinonide | 0% | 2% |
| Butylated Hydroxytoluene | 0% | 0.5% |

Methods - Two patches containing trifluoperazine and either 0% or 2% fluocinonide were applied to the upper arm of a single human subject previously sensitized to trifluoperazine. The patches were removed after two days and the sites read immediately and 24 and 48 hours later as well.

Results - The incorporation of 2% fluocinonide significantly reduced the magnitude of the allergic reaction (erythema and blistering) caused by trifluoperazine.

The results are as follows:

| Percent Fluocinonide | Time of Grading | | |
|---|---|---|---|
| | 48 Hours | 72 Hours | 96 Hours |
| 0% | 4+* | 4+ | 4+ |
| 2% | 2+ | 2+ | 1+ |

* erythema extended outside of patch area

**EXAMPLE I (f)**

PROTOCOL - TIMOLOL

Objective - To block the irritant dermatitis which occurs following application of timolol patches to the skin through the incorporation of hydrocortisone in the patches.

Materials - Two formulations of the antihypertensive drug timolol were used in this study. One formulation contained no hydrocortisone and the other contained 2% hydrocortisone.

Thus, the following formulations were prepared:

| | L528-7-A (Parts by weight) | L477-59 (Parts by Weight) |
|---|---|---|
| Timolol | 30% | 30% |
| ADMEX$^R$760 | 29% | 28% |
| Resin (96% repeating units of vinyl chloride and 4% repeating units of vinyl acetate) | 30% | 29% |
| Epoxidized Soybean Oil | 9% | 9% |
| SAG"· 5693* | 1% | 1% |
| Citric Acid | 1% | 1% |
| Hydrocortisone | 0% | 2% |

-----------------

* polypropylene glycol, dimethylpolysilane FCC, silicon dioxide manufactured by Union Carbide Corp. Danbury, Ct. 06817.

Methods - One patch of each timolol formulation. one containing no hydrocortisone and the other containing 2% hydrocortisone were applied to the upper arm of a single human subject not previously exposed to timolol. The patches were removed at 48 hours and the sites examined for signs of dermatitis immediately and 24 hours later.

Results - Signs of dermatitis were observed at the site exposed to the timolol patch containing no hydrocortisone. No dermatitis was observed at the site exposed to the hydrocortisone patch.

| Percent Hydrocortisone | Time of Grading: 48 Hours | 72 Hours |
|---|---|---|
| 0% | 2+ | 2+ |
| 2% | 0 | 0 |

In Figure 14:
The boxed information in the upper left hand portion of the drawing should read as follows:

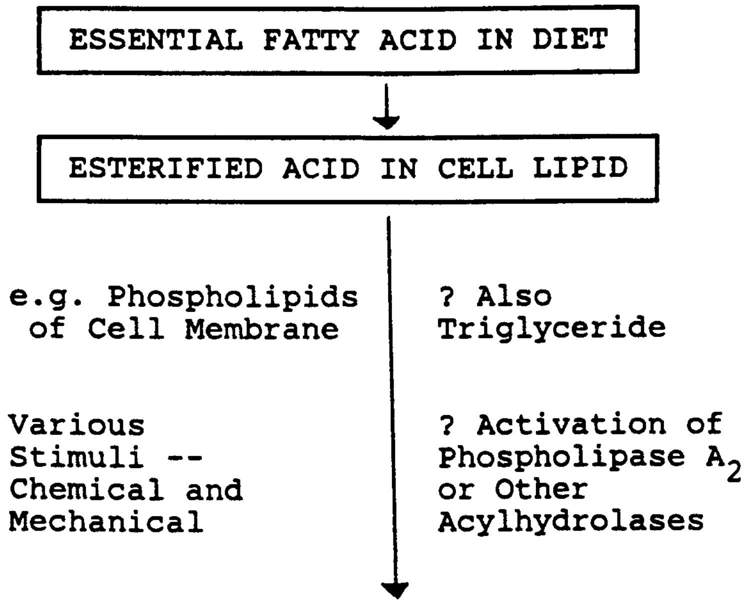


## Claims

1. A transdermal device for the controlled release of a pharmacologically active agent which is a causative factor of contact dermititis and of an anti-dermatitic substance which is chemically compatible with the pharmacologically active agent and which prevents or minimises the occurrence of said contact dermititis, characterised in that the device includes a reservoir for said pharmacologically active agent which comprises a polyvinylchloride plastisol layer having said pharmacologically active agent dispersed therein.

2. A device according to claim 1 wherein the anti-dermatitic agent is dispersed in said plastisol.

3. A device according to claim 1 or claim 2, wherein the device comprises a multi-layered structure comprising:-
   (a) a backing layer which is substantially impervious to said pharmacologically active agent,
   (b) a solid layer of said plastisol on said backing layer, and
   (c) a pressure-sensitive adhesive layer for holding the device in skin contact with the plastisol layer facing the skin.

4. A device according to claim 3, wherein a microporous membrane is disposed between the plastisol layer and the adhesive layer to regulate the rate of diffusion of the pharmacologically active agent from the reservoir.

5. A device according to any one of the preceding claims wherein the pharmacologically active agent is present in the reservoir in an amount of from 0.5 to 40% by weight.

6. A device according to claim 5, wherein the anti-dermatitic agent is present in the reservoir in an amount of from 1 to 5% by weight.

7. A device according to any one of the preceding claims, wherein the anti-dermatitic substance is selected from the group consisting of:-
   (a) corticosteroids;
   (b) chemicals that compete with the arachidonic acid biotransformation;
   (c) inhibitor substances of the arachidonic acid cascade;
   (d) free radical scavenger substances;
   (e) Vitamin E;
   (f) nordihydroguaiaretic acid;

(g) Vitamin D; and
(h) leukotriene receptor antagonists.

**8.** A device according to any one of the preceding claims, wherein the pharmacologically active substance is selected from the group consisting of:-
(a) guanfacine;
(b) (5Z, 13E, 15R, 16R)-16-fluoro-15-hydroxy-9-oxoprosta-5-dienoic acid;
(c) fluphenazine;
(d) clonidine;
(e) trifluroperazine; and
(f) timolol.

**9.** A device according to claim 7, wherein the anti-dermatitic substance is a corticosteroid and the corticosteroid is selected from the group consisting of:-
(a) hydrocortisone; and
(b) fluocinonide.

**10.** A device according to claim 7, wherein:-
(i) the anti-dermatitic substance is hydrocortisone; and
(ii) the pharmacologically active substance is guanfacine.

**Patentansprüche**

**1.** Transdermale Vorrichtung für die kontrollierte Freisetzung eines pharmakologisch wirksamen Mittels, das ein ursächlicher Faktor von Kontaktdermatitis ist, und einer Anti-dermatitis-Substanz, die mit dem pharmakologisch wirksamen Mittel chemisch kompatibel ist und das Auftreten der Kontaktdermatitis verhindert oder minimiert,
dadurch gekennzeichnet,
daß die Vorrichtung ein Reservoir für das pharmakologisch wirksame Mittel aufweist, das eine Polyvinylchlorid-Plastisolschicht aufweist, in der das pharmakologisch wirksame Mittel dispergiert ist.

**2.** Vorrichtung nach Anspruch 1, wobei das Antidermatitis-Mittel in dem Plastisol dispergiert ist.

**3.** Vorrichtung nach Anspruch 1 oder 2, wobei die Vorrichtung eine vielschichtige Struktur hat, die folgendes aufweist:
(a) eine Trägerschicht, die für das pharmakologisch wirksame Mittel im wesentlichen undurchlässig ist,
(b) eine feste Schicht des Plastisols auf der Trägerschicht und
(c) eine Kontakt-Klebstoffschicht, um die Vorrichtung in Hautkontakt zu halten, wobei die Plastisolschicht der Haut zugewandt ist.

**4.** Vorrichtung nach Anspruch 3, wobei zwischen der Plastisolschicht und der Klebstoffschicht eine mikroporöse Membran angeordnet ist, um die Diffusionsrate des pharmakologisch wirksamen Mittels aus dem Reservoir zu regeln.

**5.** Vorrichtung nach einem der vorhergehenden Ansprüche, wobei das pharmakologisch wirksame Mittel in dem Reservoir in einer Menge von 0,5-40 Gew.-% anwesend ist.

**6.** Vorrrichtung nach Anspruch 5, wobei das Antidermatitis-Mittel in dem Reservoir in einer Menge von 1-5 Gew.-% anwesend ist.

**7.** Vorrichtung nach einem der vorhergehenden Ansprüche, wobei die Antidermatitis-Substanz ausgewählt ist aus der Gruppe, die aus folgendem besteht:
(a) Corticosteroiden;
(b) Chemikalien, die mit der Arachidonsäure-Biotransformation konkurrieren;
(c) Inhibitor-Substanzen der Arachidonsäure-Kaskade;
(d) Radikal-Fängersubstanzen;
(e) Vitamin E;

(f) Nordihydroguajaretsäure;
(g) Vitamin D; und
(h) Leukotrienrezeptor-Antagonisten.

**8.** Vorrichtung nach einem der vorhergehenden Ansprüche, wobei das pharmakologisch wirksame Mittel ausgewählt ist aus der Gruppe, die aus folgendem besteht:
(a) Guanfacin;
(b) (5Z,13E,15R,16R)-16-Fluoro-15-hydroxy-9-oxoprosta-5-diensäure;
(c) Fluphenazin;
(d) Clonidin;
(e) Trifluroperazin; und
(f) Timolol.

**9.** Vorrichtung nach Anspruch 7, wobei die Antidermatitis-Substanz ein Corticosteroid ist und das Corticosteroid ausgewählt ist aus der Gruppe, die aus folgendem besteht:
(a) Hydrocortison; und
(b) Fluocinonid.

**10.** Vorrichtung nach Anspruch 7, wobei:
(i) die Antidermatitis-Substanz Hydrocortison ist; und
(ii) das pharmakologisch wirksame Mittel Guanfacin ist.

**Revendications**

**1.** Dispositif transdermique pour la libération contrôlée d'un agent pharmacologiquement actif qui est un facteur causal de la dermatite de contact et d'une substance anti-dermatite qui est chimiquement compatible avec l'agent pharmacologiquement actif et qui empêche ou réduit au minimum la survenue de ladite dermatite de contact, caractérisé en ce que le dispositif comporte un réservoir pour ledit agent pharmacologiquement actif qui comprend une couche de plastisol de polychlorure de vinyle, dans lequel ledit agent pharmacologiquement actif est dispersé.

**2.** Dispositif selon la revendication 1, dans lequel l'agent anti-dermatite est dispersé dans ledit plastisol.

**3.** Dispositif selon la revendication 1 ou 2, qui comprend une structure multicouche constituée par :
(a) une couche de renfort qui est pratiquement imperméable audit agent pharmacologiquement actif,
(b) une couche solide dudit plastisol sur ladite couche de renfort, et
(c) une couche adhésive sensible à la pression pour maintenir le dispositif en contact avec la peau, la couche de plastisol faisant face à la peau.

**4.** Dispositif selon la revendication 3, dans lequel une membrane microporeuse est disposée entre la couche de plastisol et la couche adhésive pour réguler la vitesse de diffusion de l'agent pharmacologiquement actif à partir du réservoir.

**5.** Dispositif selon l'une quelconque des revendications précédentes, dans lequel l'agent pharmacologiquement actif est présent dans le réservoir dans une proportion de 0,5 à 40 % en poids.

**6.** Dispositif selon la revendication 5, l'agent anti-dermatite est présent dans le réservoir dans une proportion de 1 à 5 % en poids.

**7.** Dispositif selon l'une quelconque des revendications précédentes, dans lequel la substance anti-dermatite est choisie parmi :
(a) les corticostéroïdes ;
(b) les produits chimiques qui entrent en compétition avec la biotransformation de l'acide arachidonique ;
(c) les substances inhibitrices de la cascade de l'acide arachidonique ;
(d) les substances fixant les radicaux libres ;
(e) la vitamine E ;
(f) l'acide nordihydroguaiarétique ;

(g) la vitamine D ; et
(h) les antagonistes des récepteurs du leukotriène.

**8.** Dispositif selon l'une quelconque des revendications précédentes, dans lequel la substance pharmacologiquement active est choisie parmi
(a) la guanfacine ;
(b) l'acide (5Z, 13E, 15R, 16R)-16-fluoro-15-hydroxy-9-oxoprosta-5-déinoïque ;
(c) la fluphénazine ;
(d) la clonidine ;
(e) la trifluropérazine ; et
(f) le timolol.

**9.** Dispositif selon la revendication 7, dans lequel la substance anti-dermatite est un cortiscostéroïde et le corticostéroïde est choisi parmi :
(a) l'hydrocortisone ; et
(b) le fluocinonide.

**10.** Dispositif selon la revendication 7, dans lequel:
(i) la substance anti-dermatite est l'hydrocortisone; et
(ii) la substance pharmacologiquement active est la guanfacine.

FIG.1

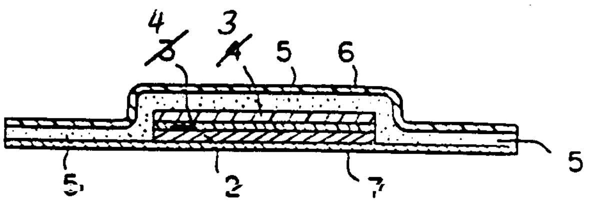


FIG.3

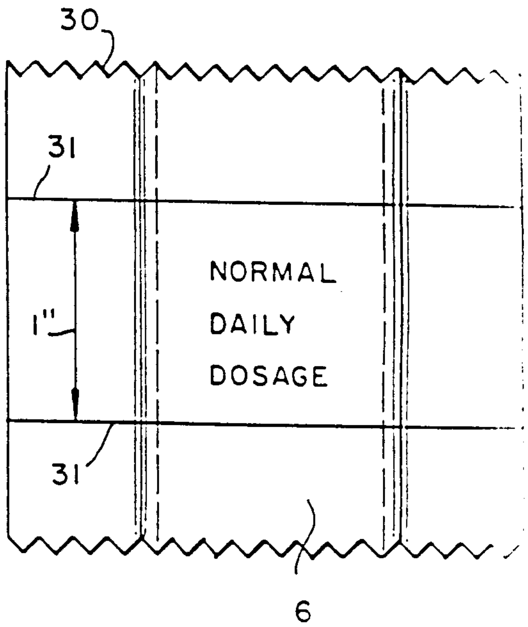


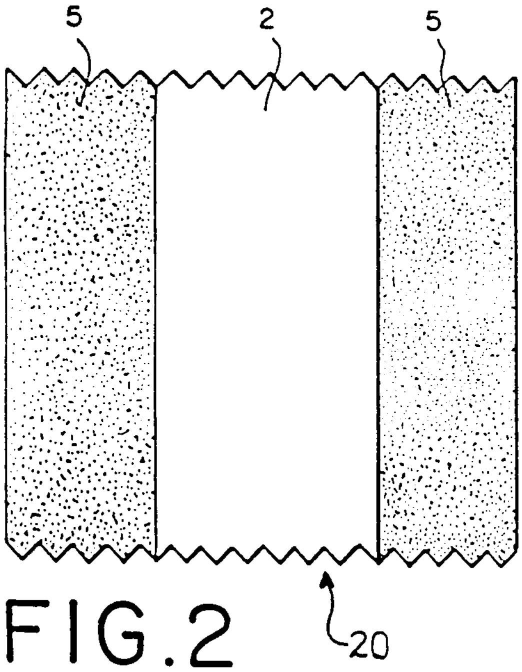


FIG.2

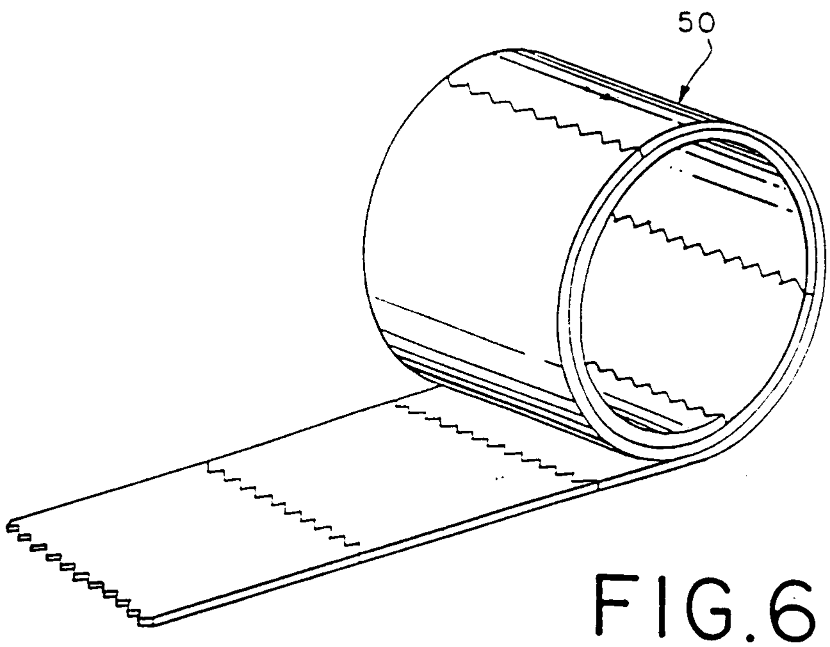


FIG.6

FIG.4

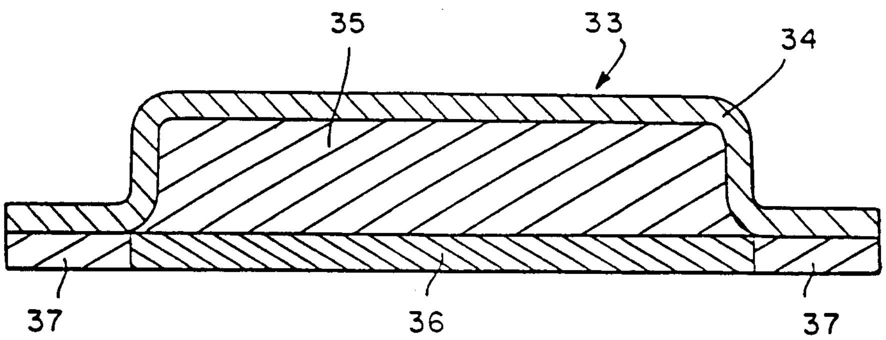


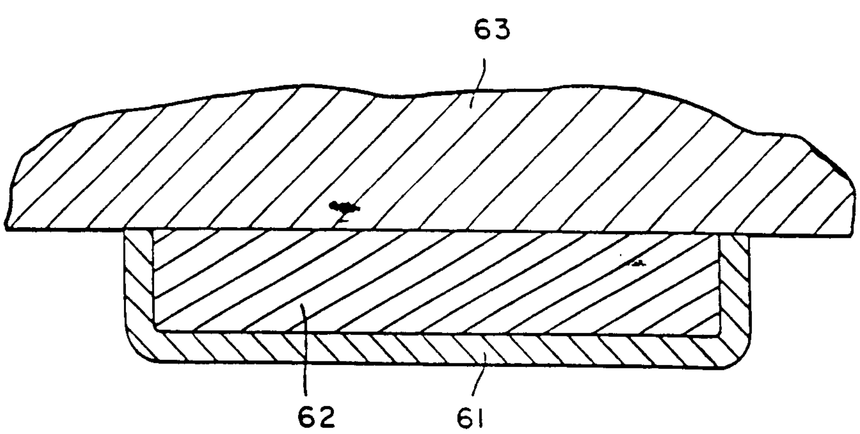


FIG.5

FIG.7

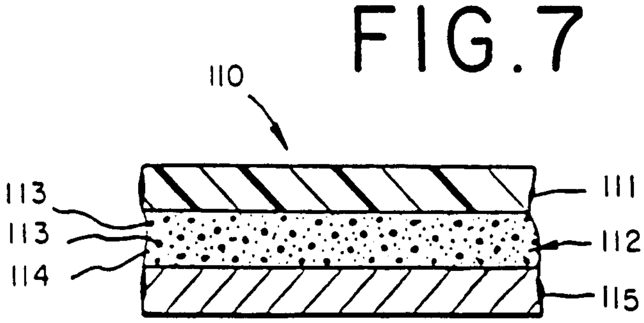


FIG.7-A

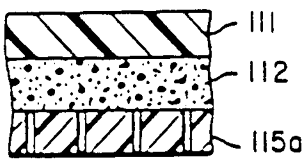


FIG.7-B

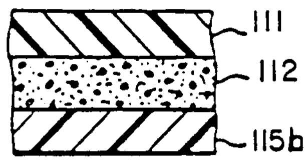


FIG.7-C

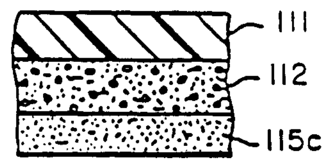


FIG.7-D

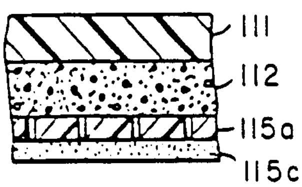


FIG.7-E

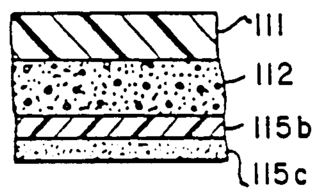


FIG.8

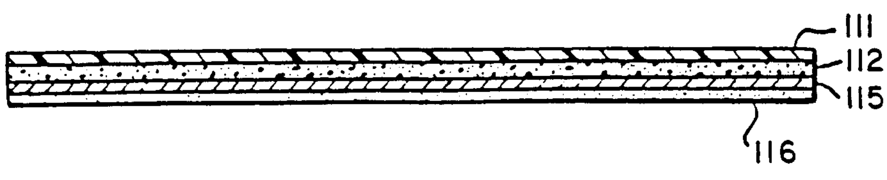


FIG.9

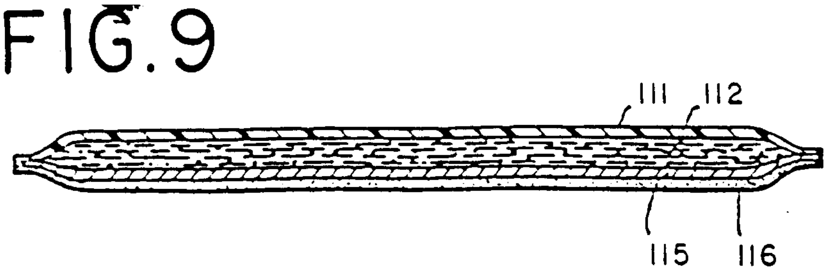

FIG.10A

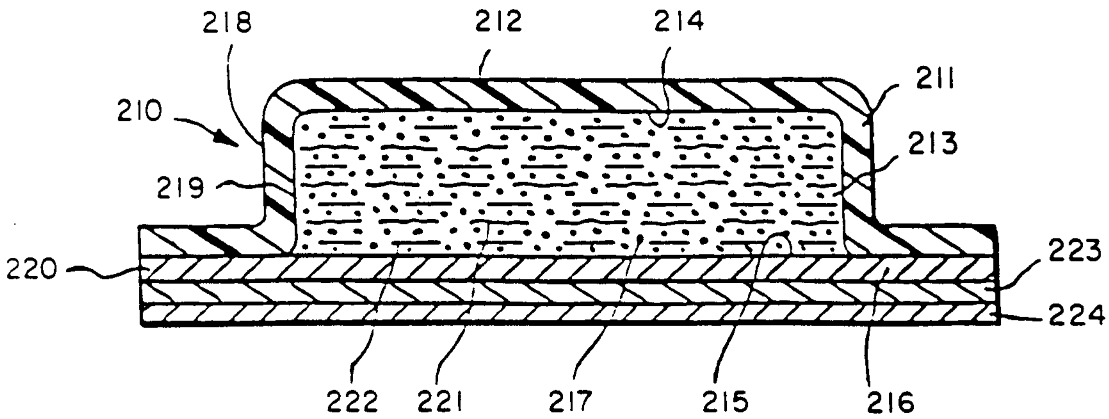


FIG.10B

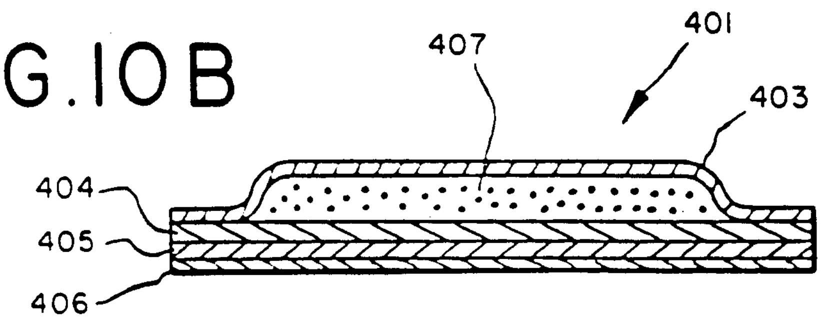


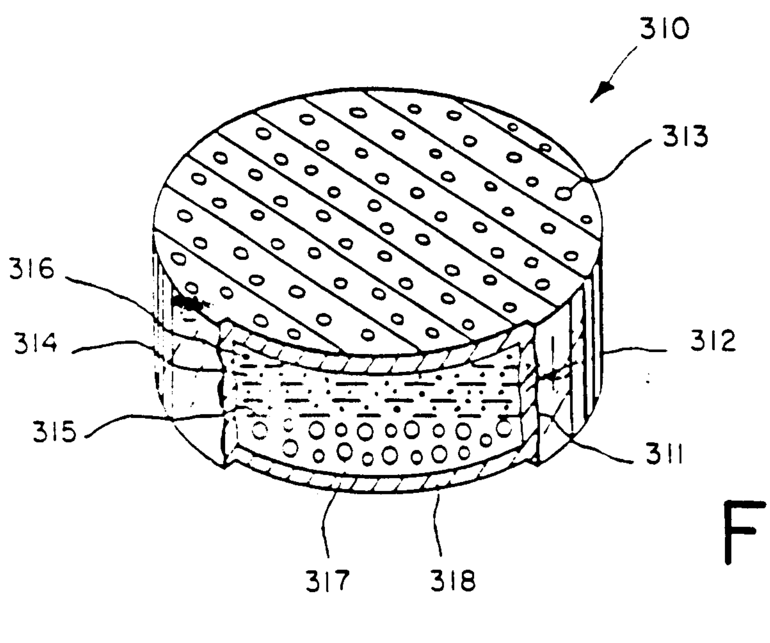


FIG.11

FIG.12

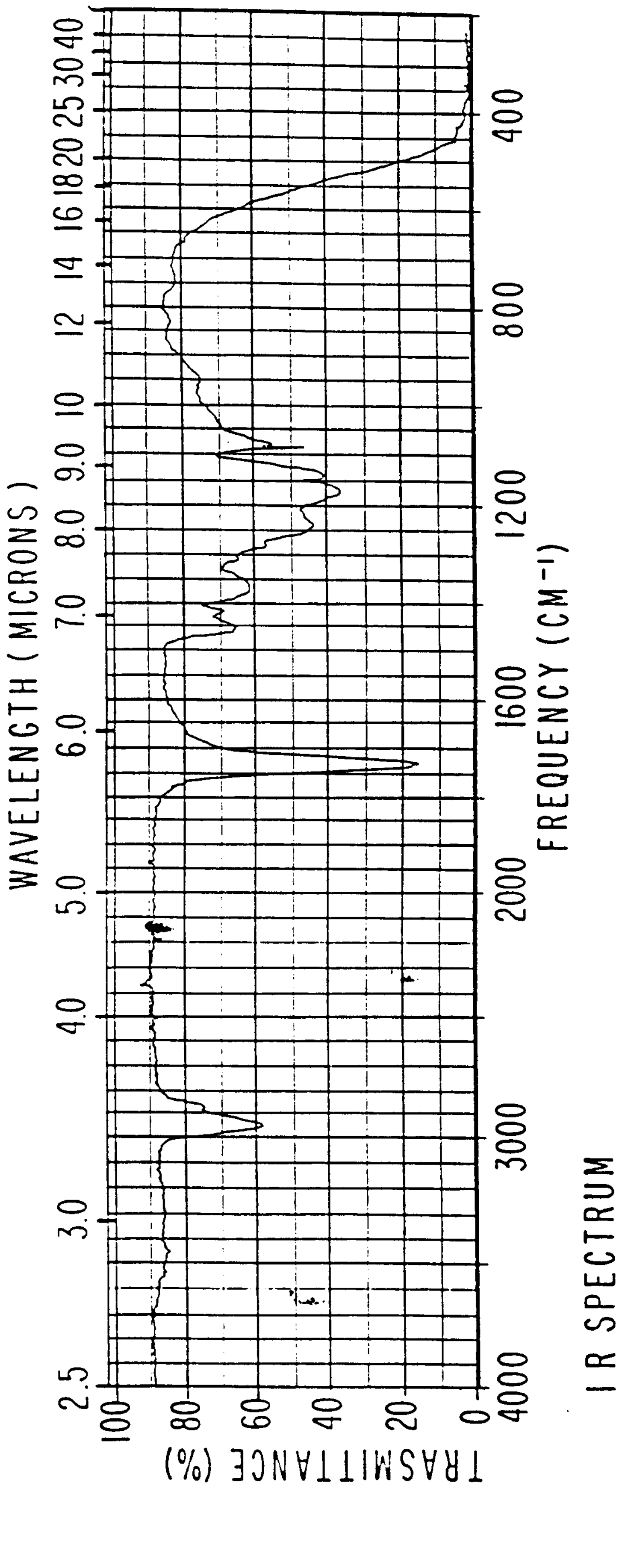
WAVELENGTH (MICRONS)
2.5
3.0
4.0
5.0
6.0
7.0
8.0
9.0
10
12
14
16
18
20
25
30
40
FREQUENCY (CM⁻¹)
4000
3000
2000
1600
1200
800
400
TRASMITTANCE (%)
100
80
60
40
20
0
I R SPECTRUM

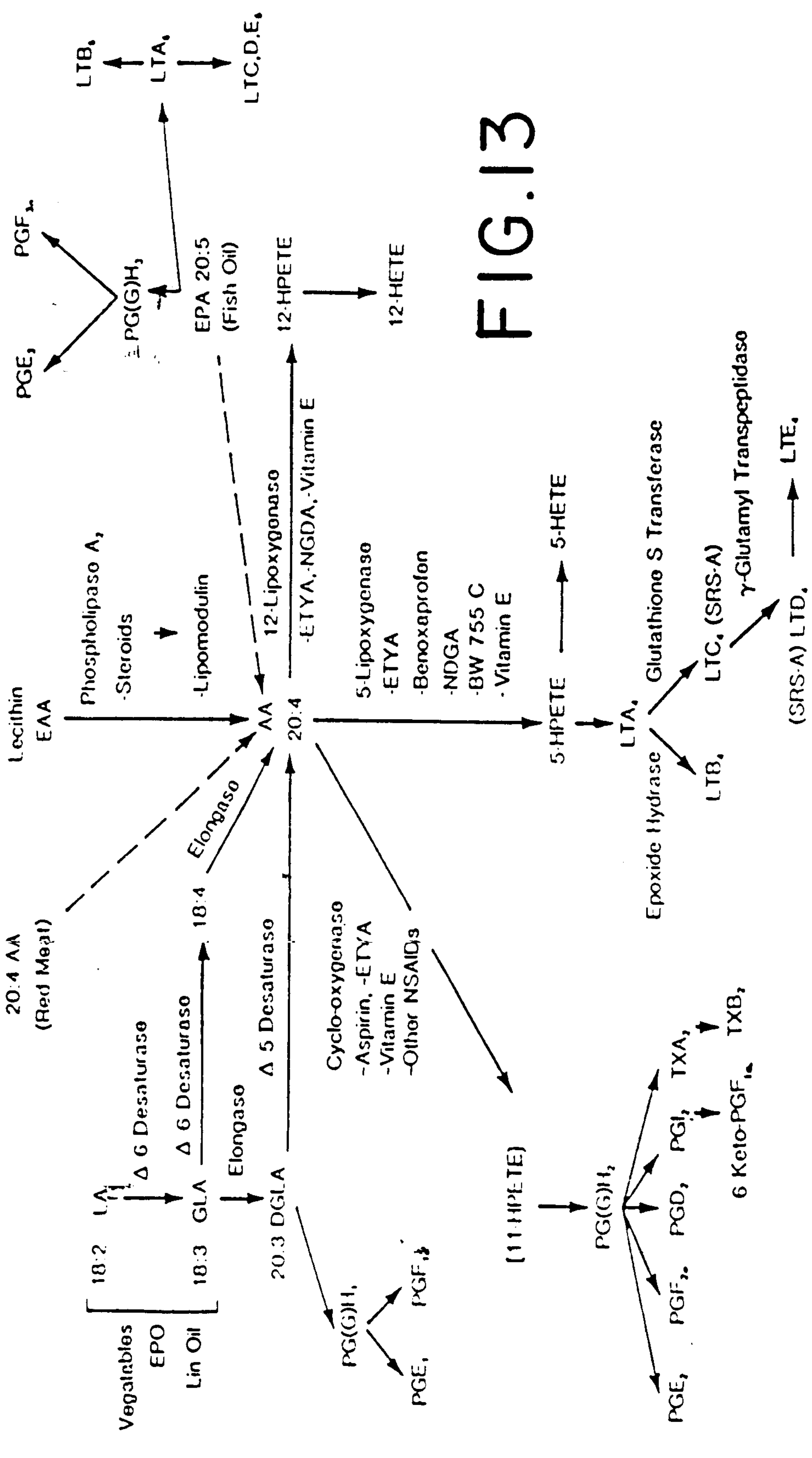
Vegetables
EPO
Lin Oil
18:2 LA
18:3 GLA
Δ 6 Desaturase
Δ 6 Desaturase
18:4
Elongase
20:3 DGLA
Δ 5 Desaturase
Elongase
20:4 AA
(Red Meat)
Lecithin
EAA
Phospholipase $A_2$
-Steroids
-Lipomodulin
AA
20:4
EPA 20:5
(Fish Oil)
12-Lipoxygenase
-ETYA,-NGDA,-Vitamin E
12-HPETE
12-HETE
5-Lipoxygenase
-ETYA
-Benoxaprofen
-NDGA
-BW 755 C
-Vitamin E
5-HPETE
5-HETE
$LTA_4$
Epoxide Hydrase
$LTB_4$
Glutathione S Transferase
$LTC_4$ (SRS-A)
γ-Glutamyl Transpeptidase
(SRS-A) $LTD_4$
$LTE_4$
Cyclo-oxygenase
-Aspirin, -ETYA
-Vitamin E
-Other NSAIDs
[11-HPETE]
$PG(G)H_2$
$PGE_2$
$PGF_{2\alpha}$
$PGD_2$
$PGI_2$
$TXA_2$
6 Keto-$PGF_{1\alpha}$
$TXB_2$
$PG(G)H_1$
$PGE_1$
$PGF_{1\alpha}$
$PG(G)H_3$
$PGE_3$
$PGF_{3\alpha}$
$LTB_5$
$LTA_5$
$LTC,D,E_5$

FIG.13

FIG.14

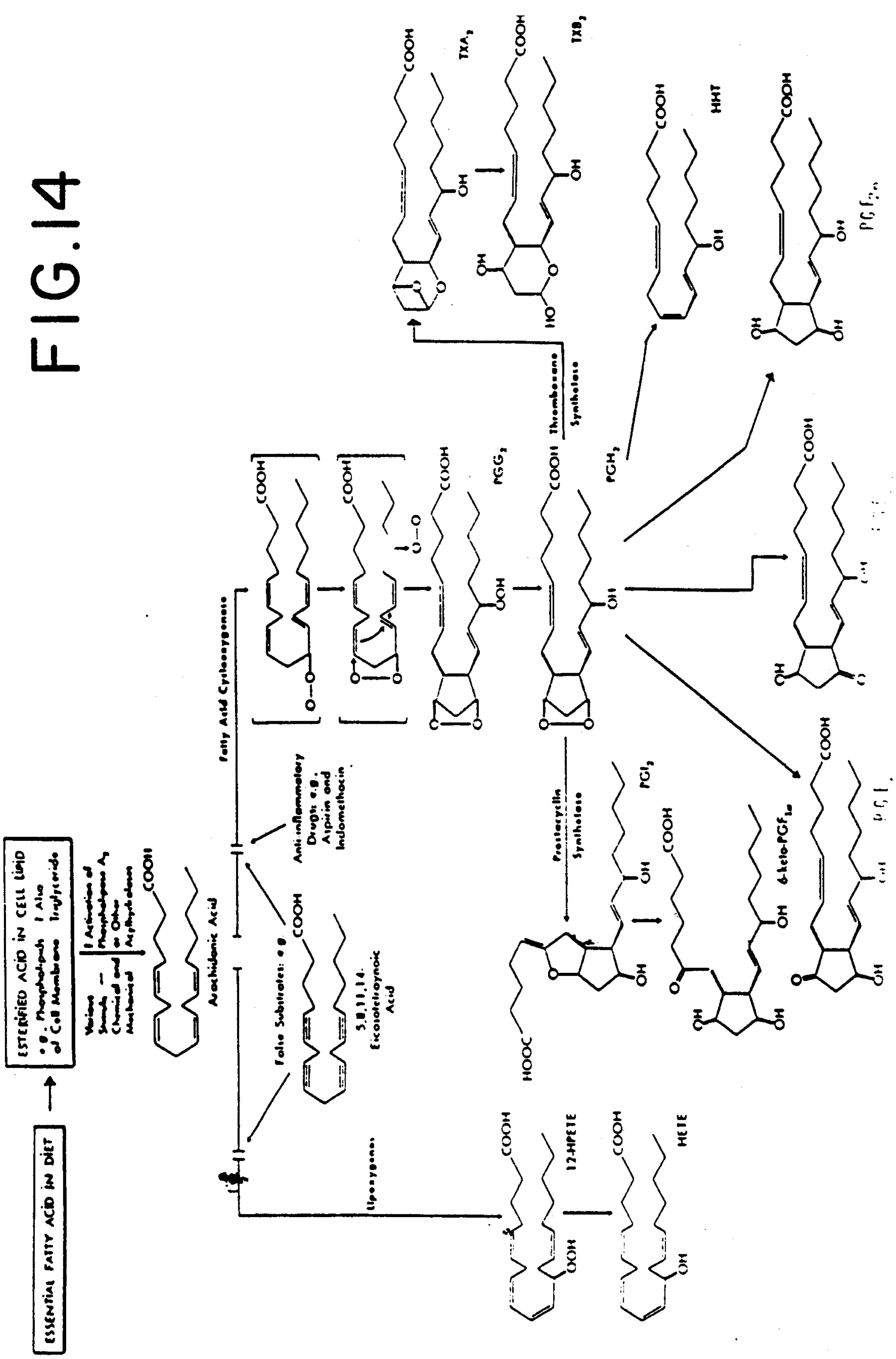
ESSENTIAL FATTY ACID IN DIET
ESTERIFIED ACID IN CELL LIPID
e.g. Phospholipids & Also of Cell Membrane Triglyceride
Various Stimuli — Chemical and Mechanical
Activation of Phospholipase A2 or Other Acylhydrolases
Arachidonic Acid
False Substrates: e.g.
5,8,11,14-Eicosatetraynoic Acid
Anti-inflammatory Drugs: e.g. Aspirin and Indomethacin
Fatty Acid Cyclooxygenase
Lipoxygenase
12-HPETE
HETE
PGG2
PGH2
Thromboxane Synthetase
TXA2
TXB2
HHT
Prostacyclin Synthetase
PGI2
6-keto-PGF1α